# EUROPEAN PATENT SPECIFICATION

(11) **EP 1 303 328 B1**
(45) Date of publication and mention of the grant of the patent: **04.10.2006**
(21) Application number: 01959138.7
(22) Date of filing: 24.07.2001
(51) Int. Cl.: A61M 16/06

(54) **Remote venting for a respiratory mask**
Entlüftung für eine Atemmaske
Aération à distance pour masque respiratoire

(30) Priority: 24.07.2000 US 624047
(43) Date of publication of application: 23.04.2003
(73) Proprietor: MALLINCKRODT INC., St. Louis, Missouri 63134 (US)
(72) Inventor: HANSEN, Gary, L., Eden Prairie, MN 55346 (US); BORDEWICK, Steven, S., Shoreview, MN 55126 (US)
(74) Representative: Hano, Christian
(86) International application number: PCT/US2001/023218
(87) International publication number: WO 2002/007807

(56) References cited:
- WO-A-00/78381
- DE-A- 19 738 266
- DE-A- 19 825 290
- US-A- 5 018 519
- US-A- 5 918 598
- US-A1- 2001 032 648

## Description

### BACKGROUND OF THE INVENTION

### 1. Field of the Invention

The present invention relates to a respiratory mask comprising the features of the preamble of claim 1.

### 2. Description of the Background Art

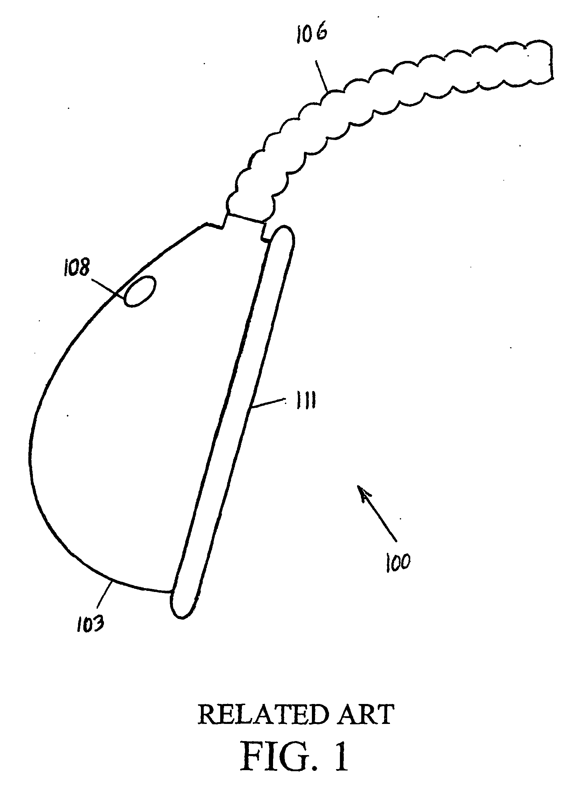

A respiratory mask is a device used to deliver a gas or gases to a person, FIG. 1 shows a respiratory mask 100 of the related art. The related art respiratory mask 100 includes a mask shell 103, a gas supply hose 106, a vent aperture 108, and an optional gasket 111. The mask shell 103 is fitted over a face of the person in order to supply a gas to a respiratory system of the person.

The respiratory mask 100 may be used to deliver any variety of gases, including air or oxygen, and a variety of medicines or treatments. Preferably, the mask 100 does not allow a supplied gas to escape. A strap or other attaching means (not shown) may be affixed to the mask shell 103 and may be fitted over the head of the person. Constant pressure gas is therefore delivered, with the vent aperture 108 maintaining a substantially constant pressure in the mask. This is referred to as a continuous positive airway pressure (CPAP) mask. The vent aperture 108 allows the exhaust of expired carbon dioxide from the mask 100. It is important that the vent aperture 108 be of a sufficient size to exhaust all exhaled air under normal conditions of use. In the related art, the need for venting has meant simply an aperture on the mask shell 103, whereby exhaled air is flushed out of the respiratory mask 100 by the positive pressure generated by the gas supply hose 106. This is taught by Rapaport U.S. Patent Re. 35,339.

Several drawbacks exist with the venting of the related art respiratory mask 100. First, the air circulation within the mask shell 103 and vent aperture 108 may create annoying noises. Second, a jet of air from the vent aperture 108 may impinge on the wearer or on nearby persons. This can be seen in FIG. 1, where the vent aperture 108 and a resulting air jet are relatively close to the face of the wearer, and will in all likelihood be in the region of persons near to or conversing with the wearer. As a result, these drawbacks may affect compliance with a therapy.

In DE 197 38 266, the air outlet is distanced from the head and neck regions and consists of a flexible outlet hose with additional air holes along the hose. The hose is fitted to the breathing mask by a fixer and once fitted the air outlet hose is guided along the compressed air hose by fixtures. The hose is made of flexible plastics and the additional air holes can be of optional diameter and arrangement so as to increase the effective diameter of the air hose.

Therefore, there remains a need in the art for an improved respiratory mask.

### SUMMARY OF THE INVENTION

A respiratory mask having a remote venting is provided according to claim1.

Preferred embodiments of the respiratory mask according to the invention are claimed in claims 2 and 3.

The above and other features and advantages of the present invention will be further understood from the following description of the preferred embodiment thereof, taken in conjunction with the accompanying drawings.

### BRIEF DESCRIPTION OF THE DRAWINGS

FIG. 1 shows a respiratory mask of the related art;
FIG. 2 shows a remotely vented respiratory mask not representing the invention;
FIG. 3 shows a further remotely vented respiratory not representing the invention;
FIGS. 4A and 4B show cross-sectional views of the gas supply hose and remote venting device of Fig. 3; and
FIG. 5 shows an embodiment of a remotely vented respiratory mask of the present invention.

### DETAILED DESCRIPTION OF THE PREFERRED EMBODIMENTS

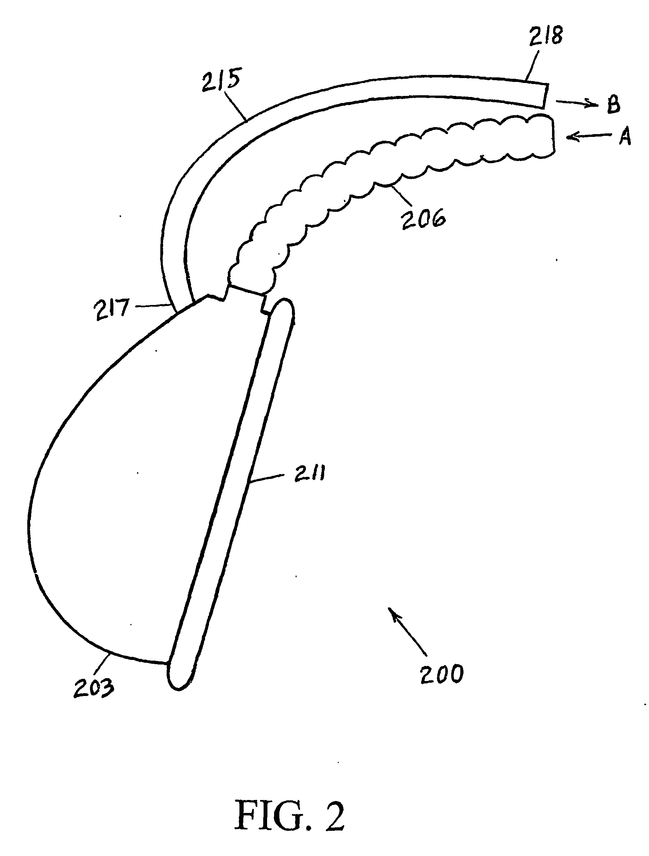

FIG. 2 shows a remotely vented respiratory mask 200 not representing the invention. The remotely vented respiratory mask 200 includes a mask shell 203, a gas supply hose 206 attached to the mask shell 203, an optional gasket 211, and a remote venting device 215. The remote venting device 215 is preferably a tube having a proximal end 217 attached to the mask shell 203 and a distal end 218 that is distant from the mask shell 203. The remote venting device 215 may be formed of a soft or rigid material. The remote venting device 215 allows a gas flow out of the mask 200 in order to exhaust expired carbon dioxide. It is estimated that a minimum gas flow volume of about ten to fifteen liters per minute at a pressure of four cm H₂O air pressure may be adequate to vent the exhaled air from the mask 200. At higher CPAP pressure, higher flow rates are acceptable. Preferably, the remote venting device 215 is designed with an internal diameter such that the resulting gas flow is adequate at the lowest CPAP working pressure, which is typically a pressure of about three to four cm H₂O. In one embodiment, the remote venting device 215 is a tube having an internal diameter of about 0.2 inch and a length of about 12 inches, producing a gas flow of about 27 liters per minute at a pressure of about 12 cm H₂O.

In use, the mask shell 203 receives a gas flow A (see arrow) from the gas supply hose 206 at an essentially constant pressure, while the remote venting device 215 allows an airflow B of exhaled air. The remote venting device 215 may be run parallel to the gas supply hose 206, and additionally may be attached to the exterior of the gas supply hose 206. As shown in the figure, it is preferred that the remote venting device 215 be run substantially parallel to the gas supply hose 206, with the distal end 218 of the remote venting device 215 being positioned as desired in order to direct the exhaled airflow B in a direction and location where it will be least offensive.

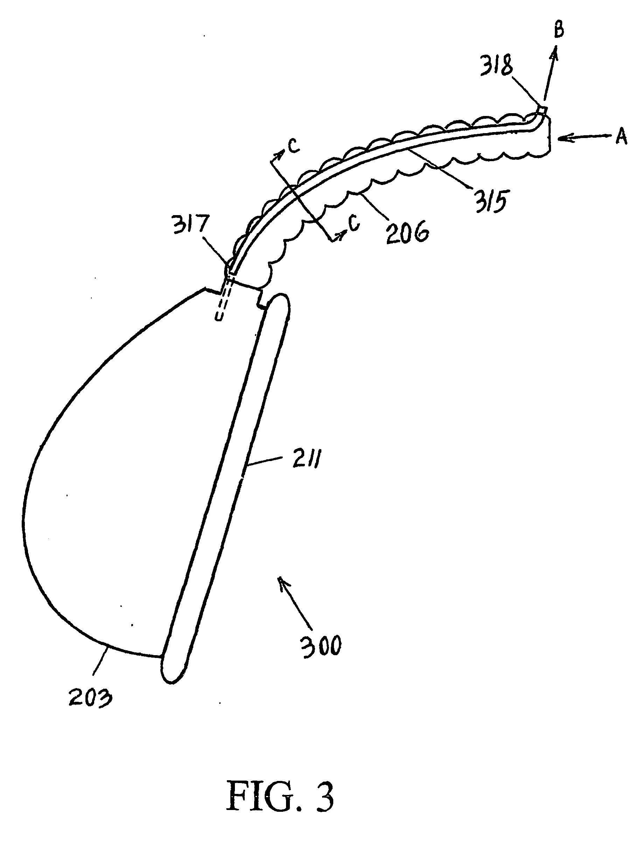

FIG. 3 shows a further remotely vented respiratory mask 300 not representing the invention. The main components are identical to the mask 200, and the reference numbers of the identical components are retained. The mask 300 includes a remote venting device 315 located internal to the gas supply hose 206. The proximal end 317 of the remote venting device 315 is preferably located in the gas supply hose 206 near the mask shell 203 (solid lines), but may alternately be located inside the mask shell 203 (dashed lines). The distal end 318 may emerge from the gas supply hose 206 at a location distant from the mask shell, as shown. Alternatively, the distal end 318 may connect to a fitting (not shown), with such a fitting including an aperture for venting exhaled air.

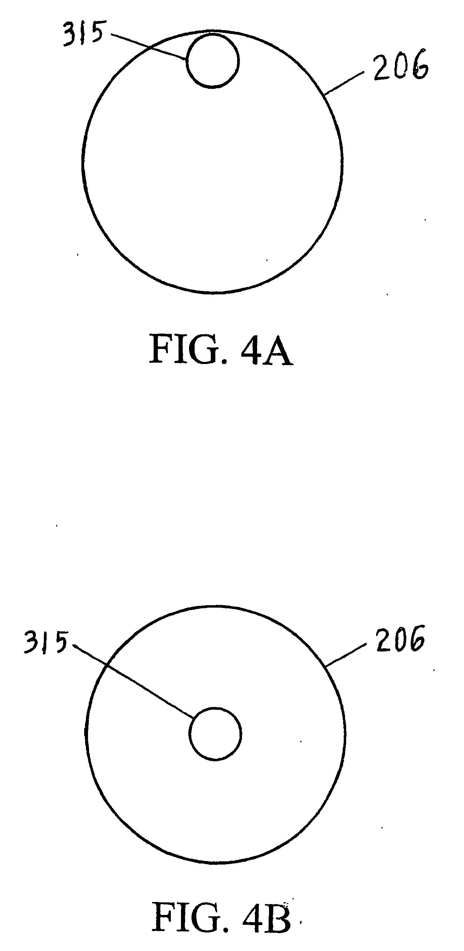

FIGS. 4A and 4B show cross-sectional views of the gas supply hose 206 and the remote venting device 315 at the section line C-C of FIG. 3. In FIG. 4A, the remote venting device 315 extends adjacent to an inner surface of the gas supply hose 206. The remote venting device 315 may be attached to an interior surface of the gas supply hose 206. In FIG. 4B, the remote venting device 315 is substantially coaxial with the gas supply hose 206.

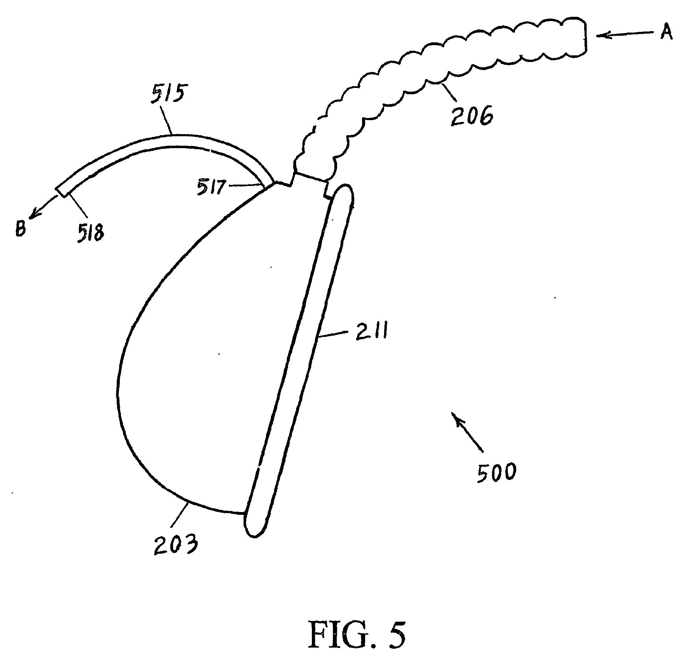

FIG. 5 shows a vented respiratory mask 500 according to the present invention. The main components are identical to the mask 200, and the reference numbers of the identical components are retained. The mask 500 includes a remote venting device 515 which does not run parallel to or near the gas supply hose 206, but instead is located away from the gas supply hose 206, and may be positioned as desired.

It should be understood that the gas supply hose 206 may run up and over the head of the respiratory mask wearer, may hang down below the respiratory mask, or may be in any other conceivable configuration. The present invention includes a remote venting device which may run with or away from the gas supply hose 206, and likewise may run up and over the head of the wearer, may hang down below the respiratory mask, or may depart from the respiratory mask in any desired direction.

The present invention results in several benefits to the wearer of the mask. The benefits include a noise reduction, a conversion of the noise into a lower region of the sound spectrum, an improved air jet direction through the placement of the remaining noise farther away from the face of the wearer, and the ability of the wearer to put the distal end of the remote venting device in any desired position.

While the invention has been described in detail above, the invention is not intended to be limited to the specific embodiment as described. It is evident that those skilled in the art may now make numerous uses and modifications of and departures from the specific embodiment described herein without departing from the scope of the invention as defined in the appended claims.

## Claims

1. A respiratory mask (500) having a remote venting, comprising:
a gas supply hose (206);
a mask shell (203) adapted to output a gas to a respiratory system of a person, said mask shell (203) adapted to be connected to said gas supply hose (206); and
a remote venting device (515) being a tube and having a proximal end (517) operably connected to an internal area of said mask shell (203) to vent carbon dioxide exhaled by said person from said mask (500), and a distal end (518), said remote venting device (515) remotely venting a gas out of said respiratory mask (500) so as to substantially reduce inhalation by said person of the exhaled carbon dioxide;
**characterized in that** said remote venting device (515) is loose from and located away from said gas supply hose (206) and is adapted to depart from said respiratory mask (500) in any desired direction.

2. The respiratory mask of claim 1, wherein said proximal end (517) of said remote venting device (515) is attached to said mask shell (203).

3. The respiratory mask of claim 1, wherein said distal end (518) of said remote venting device (515) is distant from said mask shell (203).

## Patentansprüche

1. Beatmungsmaske (500) mit einer entfernten Entlüftung, die
einen Gaszufuhrschlauch (206),
einen Maskenkorb (203), der ein Gas zu einem Atmungssystem einer Person abgeben kann, wobei der Maskenkorb (203) mit dem Gaszufuhrschlauch (206) verbunden werden kann, und
eine Fernentlüftungsvorrichtung (515) umfasst, die ein Schlauch ist und ein proximales Ende (517), das wirksam mit einem Innenbereich des Maskenkorbes (203) verbunden ist, um durch die Person ausgeatmetes Kohlendioxid aus der Maske (500) zu entlüften, und ein distales Ende (518) aufweist, wobei die Fernentlüftungsvorrichtung (515) ein Gas entfernt aus der Beatmungsmaske (500) entlüftet, um ein Einatmen des ausgeatmeten Kohlendioxids durch die Person im Wesentlichen zu reduzieren; **dadurch gekennzeichnet, dass** die Fernentlüftungsvorrichtung (515) von dem Gaszufuhrschlauch (206) lose und weg angeordnet ist und von der Beatmungsmaske (500) in irgendeine gewünschte Richtung abweichen kann.

2. Beatmungsmaske nach Anspruch 1, bei der das proximale Ende (517) der Fernentlüftungsvorrichtung (515) an dem Maskenkorb (203) befestigt ist.

3. Beatmungsmaske nach Anspruch 1, bei der das distale Ende (518) der Fernentlüftungsvorrichtung (515) sich im Abstand von dem Maskenkorb (203) befindet.

## Revendications

1. Masque respiratoire (500) présentant une aération à distance, comprenant:
un tuyau d'alimentation en gaz (206);
une coque de masque (203) adaptée pour sortir un gaz dans le système respiratoire d'une personne, ladite coque de masque (203) étant adaptée pour être connectée au dit tuyau d'alimentation en gaz (206); et
un dispositif d'aération à distance (515) étant un tuyau et présentant une extrémité proximale (517) connectée de manière fonctionnelle à une zone interne de ladite coque de masque (203) pour évacuer le dioxyde de carbone exhalé par ladite personne dudit masque (500), et une extrémité distale (518), ledit dispositif d'aération à distance (515) évacuant à distance un gaz hors dudit masque respiratoire (500) de manière à réduire substantiellement l'inhalation par ladite personne du dioxyde de carbone exhalé;
**caractérisé en ce que** ledit dispositif d'aération à distance (515) est séparé et localisé en un endroit éloigné dudit tuyau d'alimentation en gaz (206) et est adapté pour partir dudit masque respiratoire (500) dans une quelconque direction souhaitée.

2. Masque respiratoire selon la revendication 1, dans lequel ladite extrémité proximale (517) dudit dispositif d'aération à distance (515) est attachée à ladite coque de masque (203).

3. Masque respiratoire selon la revendication 1, dans lequel ladite extrémité distale (518) dudit dispositif d'aeration à distance (515) est éloignée de ladite coque de masque (203).
